# EUROPÄISCHE PATENTANMELDUNG

(11) **EP 0 605 764 A1**
(43) Veröffentlichungstag der Anmeldung: **13.07.1994**
(21) Anmeldenummer: 93117758.8
(22) Anmeldetag: 03.11.1993
(51) Int. Cl.: A61F 2/06

(54) **Instrument zum Implantieren und Extrahieren von Stents**

(30) Priorität: 08.01.1993 DE 4300285
(71) Anmelder: Richard Wolf GmbH, D-75438 Knittlingen (DE)
(72) Erfinder: Heckele, Helmut, D-75434 Knittlingen (DE)
(74) Vertreter: Wilcken, Thomas, Dipl.-Ing.

(57) **Zusammenfassung**

Das Instrument (1) besteht aus einem inneren ersten Tubus (2) und einem relativ zu diesem axial verstellbaren äußeren zweiten Tubus (3) sowie aus radial und elastisch zur Instrumentenlängsachse auslenkbaren Spreizelementen (14) zum Halten des Stents (23), wobei die Spreizelemente (11) bei in einer Richtung erfolgende Verstellung des zweiten Tubus (3) gegen den Stent (23) zur Anlage gebracht werden können. Die proximalen und distalen Enden der Spreizelemente (14) sind jeweils an einem Ring (13) befestigt und mit diesen Ringen in bezug auf ihren radialen Abstand zur Instrumentenlängsachse festgelegt. Durch Verstellung des zweiten und auf einen der Ringe (13) einwirkenden Tubus (3) kann der Abstand zwischen den Ringen (13) verändert werden, wobei bei Reduzierung dieses Abstandes die Spreizelemente (14) unter Bildung von nach außen gerichteten, kraftschlüssig gegen den Innenumfang des Stents (23) zur Anlage bringbaren Bögen verformbar sind.

## Beschreibung

Die Erfindung geht aus von einem Instrument zum Implantieren und Extrahieren von Stents zum Rekanalisieren von Hohlorganen, bestehend aus einem inneren ersten Tubus und einem relativ zu diesem axial verstellbaren äußeren zweiten Tubus sowie aus radial und elastisch zur Instrumentenlängsachse auslenkbaren Spreizelementen zum Halten des Stents, wobei die Spreizelemente bei in einer Richtung erfolgender Verstellung des zweiten Tubus gegen den Stent zur Anlage bringbar sind.

Röhrenförmige Hohlorgane neigen in Folge pathologischer Veränderungen zuweilen zur Okklusion oder Stenosierung und müssen wieder durchgängig gemacht werden. Zur Rekanalisierung werden verschiedene Verfahren angewandt, wie z.B. Laserapplikation, Kürettage oder das Einsetzen von beispielsweise tubusförmigen Prothesen, sogenannten Stents. Bei der Applikation dieser Stents treten häufig Probleme auf, weil die Stenosen sehr tief in den Hohlorganen liegen oder die Organe schon sehr stark deformiert sind. Weitere Probleme können dadurch auftreten, daß verschiedene Größen solcher Stents, entsprechend der Größe der Hohlorgane verwendet werden müssen und daher jeweils das entsprechende Instrumentarium zur Verfügung stehen muß. Des weiteren kann es erforderlich werden, während des Anbringens des Stents eine Optik verwenden und Gase oder Flüssigkeiten verabreichen zu müssen. Instrumente zur Applikation solcher Prothesen sind in vielfältiger Weise bekannt, beispielsweise aus den Schriften: US-PS 4 732 152, US-PS 4 848 343, US-PS 4 875 480, US-PS 4 553 545, US-PS 4 771 773, DE-OS 35 18 238, DE-OS 37 04 094, DE-OS 36 40 745 sowie die DE-OS 33 22 001.

Demnach sind also Instrumente bekannt, die entweder mit einem Ballon zum Aufweiten des Hohlorgans und Absetzen eines Stents versehen oder als Tubus ohne zusätzliche Spreizelemente ausgebildet sind. Während bei Ballonen häufig nachteilig ist, daß sie sehr leicht verformbar sind und sich insgesamt weniger zur Applikation von röhrenförmigen Stents eignen, bedingen die tubusförmigen Instrumente die Verwendung von Zusatzinstrumenten, wie Zangen und dergleichen zur Applikation des Stents. Eine Extraktion von Stents ist mit diesen Instrumenten nicht möglich.

Aus der EP-A1 0 364 420 ist weiter ein Instrument bekannt, mit dem es möglich ist, einen im wesentlichen tubusförmigen und radial expandierbaren Stent transluminal zu implantieren oder zu extrahieren. Mit diesem Instrument soll die Aufgabe gelöst werden, einen Stent exakt plazieren und ebenso exakt wieder extrahieren zu können, wobei beide Vorgänge unter Sicht durch Verwendung einer Optik erfolgen können. Dieses Instrument besteht im wesentlichen aus einem Innentubus, der von einem weiteren Tubus umgeben ist, welcher relativ zum inneren Tubus bewegt werden kann. Außerdem sind an dem inneren Tubus eine Vielzahl von Spreizelementen angeordnet, durch die ein Stent auf dem Instrument gehalten werden und an den Applikationsort verbracht werden kann. Der Stent wird bei zurückgeschobenem Außentubus unter die dabei radial nach außen abstehenden Spreizelemente gebracht und sodann durch Vorwärtsschieben des Außentubus radial nach innen gedrückt, wobei der Stent jeweils zwischen Außentubus und Innentubus durch die Spreizelemente festgelegt ist. Sodann wird mit Hilfe einer Optik unter Sicht der Stent an den Behandlungsort gebracht und dort plaziert, indem der oben geschilderte Vorgang zum Festhalten des Stents umgekehrt durchgeführt wird. Anschließend wird das Instrument aus der Körperhöhle wieder entfernt. Bei diesem Instrument kann auch der Raum zwischen Außen- und Innentubus dazu benutzt werden, eine Flüssigkeit in die Körperhöhle zu leiten, um das distale Ende der Endoskopoptik zur besseren Sicht freizuspülen. Ebenso können mit diesem Instrument unter Sicht Stents wieder entfernt werden, indem die vorstehend geschilderte Handhabung in umgekehrter Richtung erfolgt.

Dieses Instrument weist keine eigenen Mittel zur Beleuchtung der Körperhöhle auf und ist daher nur in Verbindung mit einem Endoskop, z.B. einem Bronchoskop, verwendbar. Auch hat dieses Instrument den gravierenden Nachteil, daß sich die freien Enden der Spreizelemente in das umliegende Gewebe spießen können, was zu schlimmen Verletzungen des Hohlorgans, beispielsweise bei der Bronchoskopie der Luftröhre und Bronchien, fuhren kann. Als weiterer Nachteil erweist sich, daß der Stent bei manchen Anwendungen nicht korrekt appliziert werden kann. Ähnliche Probleme ergeben sich auch bei Instrumenten, wie sie in DE-G 92 07 941 offenbart sind.

Es ist die Aufgabe der Erfindung, ein Instrument anzugeben, mit dem in röhrenförmigen Hohlorganen unterschiedlicher Größe Stents auf einfache Weise korrekt plaziert und ebenso einfach aus der Körperhöhle wieder entfernt werden können, und zwar so, daß eine Beeinträchtigung umliegenden Gewebes durch den Mechanismus zur Applikation des Stents ausgeschlossen ist und daß darüber hinaus die sonst üblichen endoskopischen Behandlungen von Hohlorganen mit Hilfsinstrumenten und Spülflüssigkeiten durchgeführt werden können.

Diese Aufgabe wird erfindungsgemäß dadurch gelöst, daß beim einleitend erwähnten Instrument die proximalen und distalen Enden der Spreizelemente jeweils an einem Ring befestigt und in bezug auf ihren radialen Abstand zur Instrumentenlängsachse festgelegt sind und daß durch Verstellung des zweiten, auf einen der Ringe einwirkenden Tubus der Abstand zwischen den Ringen veränderbar ist und die Spreizelemente unter Bildung von nach außen gerichteten, kraftschlüssig gegen den Innenumfang des Stents zur Anlage bringbaren Bögen verformbar sind.

Der entscheidende Vorteil eines derartig ausgebildeten Instruments besteht darin, daß aufgrund der Festlegung der proximalen und distalen Enden der Spreizelemente innerhalb der Peripherie des äußeren Tubus freie Enden der Spreizelemente vermieden sind, die sonst beim Einführen des Instruments in oder Entfernen aus einem zu rekanalisierenden Hohlorgan zu Verletzungen führen könnten. Außerdem ist die Kraft zum Halten und Verformen des Stents je nach Bedarf dosierbar, indem der zweite Tubus mehr oder weniger weit axial verstellt wird.

Nach einer bevorzugten Ausgestaltung des erfindungsgemäßen Instruments weist der innere Tubus an seinem distalen Endbereich eine Verdickung auf, deren Außendurchmesser dem des äußeren Tubus und der Ringe entspricht. Damit präsentiert sich das Instrument mit einer Spreizvorrichtung, die stufenlos in das Instrument integriert ist.

Durch Anordnung von Scheiben aus gleitfähigem Material zwischen den Ringen und der proximalen Stirnfläche der Verdickung bzw. der distalen Stirnfläche des äußeren Tubus kann der innere Tubus des Instrumentes leicht relativ zur Spreizvorrichtung und damit zum Stent verdreht werden, wodurch es möglich ist, während des Festlegens des Stents im Hohlorgan z.B. den exakten Sitz des Stents über eine in den inneren Tubus eingeführte Optik aus verschiedenen Sichtwinkein zu kontrollieren.

Eine vorteilhafte Ausführung der Spreizvorrichtung ergibt sich, wenn die Spreizelemente als Blattfedern aus einem streifenförmigen Federmaterial ausgebildet sind und ihre proximal- und distalseitige Anbindung an die Ringe jeweils über ein Gelenk erfolgt, das eine in bezug auf die Ringe und die Tuben bogenförmige Auslenkung der Blattfedern erlaubt. Dabei ergibt eine Anbindung der Spreizelemente an die Ringe über Gelenkachsen, die jeweils innerhalb des Außendurchmessers der Ringe liegen, eine Vorzugsrichtung für die Auslenkung der Spreizelemente bei Verschiebung der Ringe aufeinander zu, die radial nach außen gerichtet ist.

Der innere Tubus kann proximalseitig mit einem Aufnahmeteil verbindbar sein, der ein Einleiten von Fluiden durch den Hohlraum des inneren Tubus hindurch in das Hohlorgan ermöglicht und mit einem Anschluß zum Einführen der Optik in den Hohlraum dieses Tubus versehen sein. Damit ist es möglich, die Position des Stents zu kontrollieren und wenn nötig, z.B. bei sich verzweigenden Hohlorganen, einen Abzweig zu betrachten und die Stelle exakt aufzufinden, an welcher der Stent appliziert werden muß.

Der Aufnahmeteil kann zweckmäßigerweise auch mit einem Verstellmechanismus für die axiale Verstellung des äußeren Tubus relativ zum inneren Tubus ausgestattet sein. Dieser Mechanismus kann aus einer Verstellmutter bestehen, die mit dem proximalen Ende des äußeren Tubus in Wirkverbindung steht. Für eine Kontrolle des Verstellweges der beiden Tuben zueinander, d.h. auch des Grades der Auswölbung der Spreizelemente der Spreizvorrichtung, kann der äußere Tubus im Bereich der Verstellmutter mit einer Skala ausgestattet sein, die darüber hinaus auch eine Reproduktion von vorab als günstig ermittelten Einstellwerten ermöglicht.

Schließlich kann zur Beleuchtung des Aktionsbereiches im Hohlraum des inneren Tubus ein Lichtleiter festgelegt sein, der vom distalen Ende des Tubus bis in den proximalseitigen Aufnahmeteil mit einem Lichtleitkabelanschlußstutzen verläuft.

Ein erfindungsgemäßes Instrument wird nachstehend anhand eines in der Zeichnung dargestellten Ausführungsbeispiels näher erläutert. Es zeigen:
- Figur 1: eine Gesamtansicht des Instruments,
- Figur 2: das Instrument nach Figur 1 im Längsschnitt und partiell vergrößert dargestellt,
- Figur 3: eine vergrößerte Längsschnittdarstellung des distalen Bereiches des Instruments,
- Figur 4: eine vergrößerte Detail-Schnittansicht eines Spreizelementes,
- Figur 5: einen Ring der Spreizvorrichtung mit angelenkten Spreizelementen,
- Figur 6: eine Stirnansicht der Ausführung nach Figur 5,
- Figur 7: eine Darstellung entsprechend Figur 3 mit einem die Spreizvorrichtung umschließenden Stent und
- Figur 8: eine Darstellung entsprechend Figur 7 mit einem Stent in expandierter Stellung.

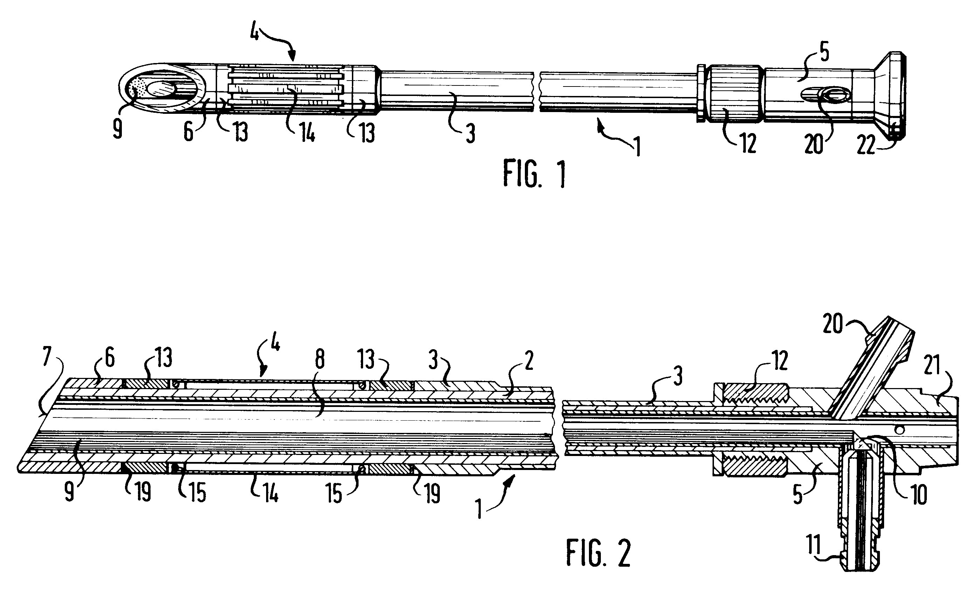

Das Instrument 1 besteht gemäß Figuren 1 und 2 im wesentlichen aus einem inneren Tubus 2 und einem zu diesem relativ verdrehbaren, axial verstellbaren und wahlweise feststellbaren äußeren Tubus 3, einer Spreizvorrichtung 4 im distalen Bereich und einem Aufnahmeteil 5 am proximalen Ende des Instruments.

Der innere Tubus 2 ist an seinem proximalen Ende in dem Aufnahmeteil 5 lösbar festgelegt und distal mit einer Verdickung 6 versehen, die in einer Anschrägung 7 endet. In dem Hohlraum 8 des inneren Tubus 2 ist weiter ein Bündel von Lichtleitern 9 festgelegt, die proximalseitig vor einem Prisma 10 enden. Ein Lichtleiterkabelanschlußstutzen 11 gehört zum Aufnahmeteil 5. Distalseitig schließen die Lichtleiter 9 mit dem inneren Tubus 2 ab.

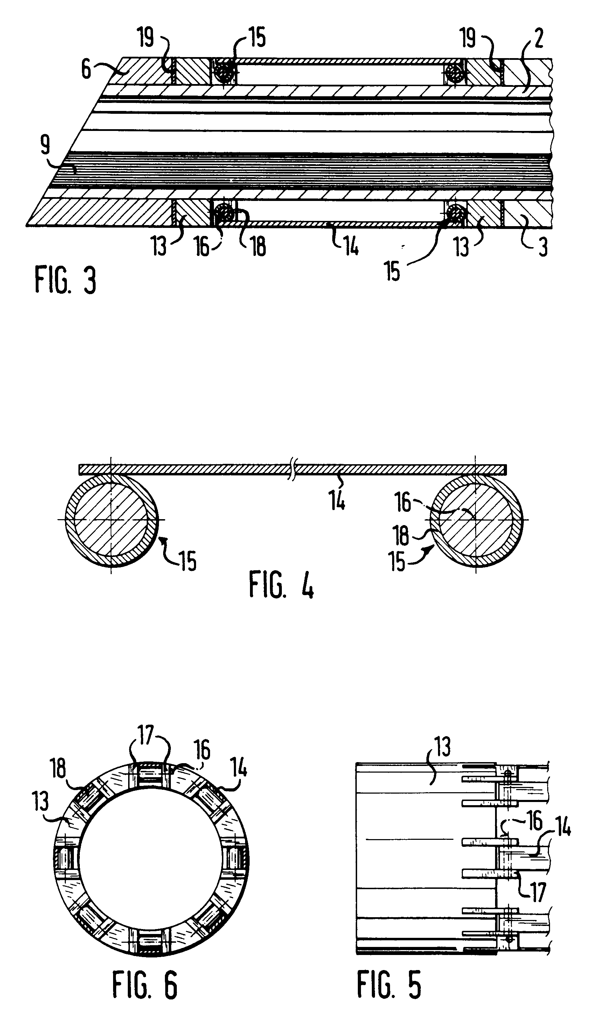

Der äußere Tubus 3 ist frei verschiebbar auf dem inneren Tubus 2 gelagert und stützt sich proximalseitig gegen eine Verstellmutter 12 ab. Der äußere Tubus 3 ist gegenüber dem inneren 2 verkürzt, so daß zwischen der proximalen Stirnfläche der Verdickung 6 und der distalen Stirnfläche des äußeren Tubus 3 ein Freiraum gebildet wird. In diesem Freiraum ist die Spreizvorrichtung 4 angeordnet (siehe auch Figur 3). Sie umfaßt zwei Ringe 13, die gleitfähig auf dem inneren Tubus 2 geführt und durch Spreizelemente 14 miteinander verbunden sind. Die Spreizelemente 14 sind als Blattfedern aus einem streifenförmigen Federmaterial ausgebildet und mit ihren Enden gelenkig mit den Ringen 13 verbunden, wodurch ein Lamellenrohrkörper gebildet wird. Die Gelenke 15 werden gemäß Figuren 4, 5 und 6 jeweils durch eine Gelenkachse 16 gebildet, die einen aus der Stirnseite jedes Ringes 13 herausragenden Gabelkopf 17 durchsetzt. Auf den Gelenkachsen 16 ist jeweils ein Rohr 18 gelagert, an dessen Mantelfläche das Ende einer der besagten Blattfedern unter tangentialer Ausrichtung in geeigneter Weise durch z.B. Kleben, Löten oder Schweißen befestigt ist.

Die Ringe 13 stützen sich jeweils mit ihrer freien Stirnseite gegen Scheiben 19 aus einem reibungsarmen Werkstoff ab, die zwischen den Ringen 13 und der proximalseitigen Stirnfläche der Verdickung 6 bzw. der distalseitigen Stirnfläche des äußeren Tubus 3 angeordnet sind.

Wie weiter insbesondere aus Figur 2 erkennbar ist, weist der Aufnahmeteil einen zusätzlichen Anschlußstutzen 20 auf, der mit dem Hohlraum 8 des inneren Tubus 2 in Verbindung steht und der Einleitung von Fluiden durch den Hohlraum 8 hindurch in das Hohlorgan dienen können. Weiter ist ein Anschluß 21 vorgesehen, auf dem eine durch den Hohlraum 8 des inneren Tubus 2 hindurch führbare Optik 22 lösbar festgelegt werden kann. Durch den zwischen innerem Tubus 2 und Optik 22 verbleibenden Freiraum können Hilfsinstrumente und die erwähnten Fluide hindurchgeführt werden.

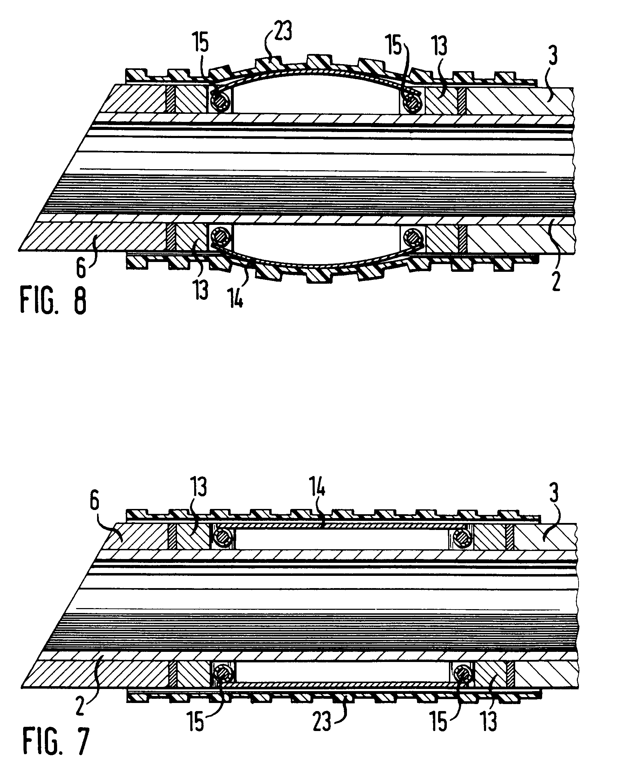

Bei der Anwendung des erfindungsgemäßen Instruments 1 wird in folgender Weise vorgegangen: Ein zum Einsetzen in ein Hohlorgan passend ausgewählter Stent 23 wird zunächst über die Verdickung 6 des inneren Tubus 2 hinweg über die Spreizelemente 14 geschoben (Figur 7). Sodann wird durch Drehen der Verstellmutter 12 der äußere Tubus 3 auf dem inneren Tubus 2 distalwärts vorgeschoben, wodurch sich der Abstand zwischen den Ringen 13 verringert mit der Folge, daß die Spreizelemente 14 sich radial auswölben und kraftschlüssig gegen die Innenfläche des Stents zur Anlage kommen (Figur 8). Auf diese Weise wird der die Spreizelemente 14 umhüllende Stent 23 an dem Instrument 1 festgelegt. Sodann wird unter visueller Beobachtung mittels der Optik 22 das Instrument 1 mit dem Stent in das Hohlorgan eingeführt und dieser an den zu behandelnden Ort verbracht. Dort werden durch entsprechende Rückdrehung der Verstellmutter 12 die Spreizelemente 14 in ihre Ruhelage zurückgeführt, so daß das Instrument 1 aus dem plazierten Stent herausgezogen werden kann, während dieser an dem Behandlungsort verbleibt.

Bei diesem Vorgehen erweist sich als vorteilhaft, daß der innere Tubus 2 gegenüber dem Stent 23 verdreht werden kann, denn dadurch ist es möglich, den Stent 23 bezüglich seines Sitzes unter Sicht aus verschiedenen Blickwinkeln zu kontrollieren und den Sitz des Stents nach erneuter Verbindung mit dem Instrument zu korrigieren.

Mit dem erfindungsgemäßen Instrument 1 ist selbstverständlich auch das Entfernen eines Stents 23 möglich. Dazu ist lediglich erforderlich, die vorstehend beschriebene Vorgehensweise umzukehren.

Die Stärke der Spreizung der Spreizelemente 14 kann mit einer nicht gezeigten Skala überwacht werden, die den Verschiebeweg des äußeren Tubus 3 und damit das Spreizmaß der Spreizelemente 14 anzeigt. Dadurch kann die Gefahr praktisch ausgeschlossen werden, daß das Hohlorgan und der Stent 23 selbst überdehnt werden.

Die beschriebene Verstellung des äußeren Tubus relativ zum inneren läßt sich konstruktiv besonders einfach lösen. Hinsichtlich der Funktion des Instrumentes kommt man aber auch zu gleichen Ergebnissen, wenn der innere Tubus relativ zum dann feststehenden äußeren Tubus verstellbar ist.

Im übrigen kann das Instrument auch zu anderen Zwecken verwendet werden, also nicht nur zum Inplantieren und Extrahieren von Stenz. So ist es beispielsweise möglich, mit den Spreizelementen Hohlorgane und Körperkanäle aufzuweiten, um Stenosen zu beseitigen, oder Gewebe, wie etwa die Bauchdecke, und Organe anzuheben bzw. von einem Ort im Körper wegzuhalten, an dem eine Diagnose oder Therapie durchgeführt werden soll.

## Patentansprüche

1. Instrument (1) zum Implantieren und Extrahieren von Stents (23) zum Rekanalisieren von Hohlorganen, bestehend aus einem inneren ersten Tubus (2) und einem relativ zu diesem axial verstellbaren äußeren zweiten Tubus (3) sowie aus radial und elastisch zur Instrumentenlängsachse auslenkbaren Spreizelementen (14) zum Halten des Stents (23), wobei die Spreizelemente (14) bei in einer Richtung erfolgender Verstellung des zweiten Tubus (3) gegen den Stent (23) zur Anlage bringbar sind, dadurch gekennzeichnet, daß die proximalen und distalen Enden der Spreizelemente (14) jeweils an einem Ring (13) befestigt und in bezug auf ihrem radialen Abstand zur Instrumentenlängsachse festgelegt sind und daß durch Verstellung des zweiten, auf einen der Ringe (13) einwirkenden Tubus (3) der Abstand zwischen den Ringen (13) veränderbar ist und die Spreizelemente (14) unter Bildung von nach außen gerichteten, kraftschlüssig gegen den Innenumfang des Stents (23) zur Anlage bringbaren Bögen verformbar sind.

2. Instrument nach Anspruch 1, dadurch gekennzeichnet, daß der innere Tubus (2) an seinem distalen Endbereich eine Verdickung (6) mit einem dem Außendurchmesser des äußeren Tubus (3) entsprechenden Außendurchmesser aufweist und daß die Ringe (13) zwischen der Verdickung (6) und der distalen Stirnfläche des äußeren Tubus (3) angeordnet sind.

3. Instrument nach Anspruch 1 oder 2, dadurch gekennzeichnet, daß die Ringe (13) auf dem inneren Tubus (2) frei drehbar gelagert sind und ihr Außendurchmesser dem des äußeren Tubus (3) entspricht.

4. Instrument nach Anspruch 3, dadurch gekennzeichnet, daß jeweils zwischen den Ringen (13) und der proximalseitigen Stirnfläche der Verdickung (6) bzw. der distalseitigen Stirnfläche des äußeren Tubus (3) Scheiben (19) aus einem reibungsarmen Werkstoff angeordnet sind.

5. Instrument nach einem der Ansprüche 1 bis 4, dadurch gekennzeichnet, daß die Spreizelemente (14) als Blattfedern aus einem streifenförmigen Federmaterial ausgebildet sind und ihre proximalseitige und distalseitige Anbindung an die Ringe (13) jeweils über ein Gelenk (15) erfolgt, das eine in bezug auf die Ringe (13) radiale Auslenkung der Spreizelemente erlaubt.

6. Instrument nach einem der Ansprüche 1 bis 5, dadurch gekennzeichnet, daß die Spreizelemente (14) mit den Ringen (13) ein Lamellenrohr bilden, dessen Außendurchmesser dem des äußeren Tubus (3) und dem der Verdickung (6) entspricht.

7. Instrument nach Anspruch 5 oder 6, dadurch gekennzeichnet, daß die Gelenkachsen (16) der Spreizelemente (14) jeweils innerhalb des Außendurchmessers des Lamellenrohres angeordnet sind.

8. Instrument nach einem der Ansprüche 1 bis 7, dadurch gekennzeichnet, daß der innere Tubus (2) proximalseitig mit einem Aufnahmeteil (5) verbindbar ist, das mit mindestens einem Anschlußstutzen (20) zur Einleitung von Fluiden in den Hohlraum (8) des inneren Tubus (2) versehen ist.

9. Instrument nach Anspruch 8, dadurch gekennzeichnet, daß das Aufnahmeteil (5) an seiner proximalen Stirnseite mit einem Anschluß (21) zum Einführen einer Optik (22) in den Hohlraum (8) des inneren Tubus (2) ausgebildet ist.

10. Instrument nach einem der Ansprüche 1 bis 9, dadurch gekennzeichnet, daß die axiale Verstellung der Tuben (2, 3) zueinander durch eine Verstellmutter (12) erfolgt, die am proximalen Ende des äußeren Tubus (2) angeordnet ist.

11. Instrument nach Anspruch 10, dadurch gekennzeichnet, daß der äußere Tubus (3) im Bereich der Verstellmutter (12) mit einer Skala zur Anzeige des Verschiebeweges versehen ist.

12. Instrument nach einem der Ansprüche 1 bis 10, dadurch gekennzeichnet, daß in dem Hohlraum (8) des inneren Tubus (2) ein Lichtleiter (9) festgelegt ist, der vom distalen Ende dieses Tubus (2) bis in den proximalseitigen Aufnahmeteil (5) mit einem Lichtleiterkabelanschlußstutzen (11) verläuft.

13. Instrument nach einem der Ansprüche 8 bis 12, dadurch gekennzeichnet, daß der innere Tubus (2) und der Aufnahmeteil (5) trennbar miteinander verbunden sind.
